# EUROPÄISCHE PATENTSCHRIFT

(11) **EP 0 053 792 B1**
(45) Veröffentlichungstag und Bekanntmachung des Hinweises auf die Patenterteilung: **29.06.1983**
(21) Anmeldenummer: 81110066.8
(22) Anmeldetag: 02.12.1981
(51) Int. Cl.: C07C 29/32, C07C 31/08, C07C 31/10

(54) **Verfahren zur Herstellung von Ethanol und n-Propanol aus Methanol**
Process for synthesizing ethanol and n-propanol from methanol
Procédé de synthèse d'éthanol et de n-propanol à partir du méthanol

(30) Priorität: 10.12.1980 DE 3046481
(43) Veröffentlichungstag der Anmeldung: 16.06.1982
(73) Patentinhaber: Ruhrchemie Aktiengesellschaft, D-4200 Oberhausen 13 (DE)
(72) Erfinder: Cornils, Boy, Dr. Dipl-Chem, D-4220 Dinslaken (DE); Frohning, Carl Dieter, Dr. Dipl.-Chem., D-4200 Oberhausen 13 (DE); Diekhaus, Gerhard, Dr. Dipl.-CHem, D-4200 Oberhausen 14 (DE); Wiebus, Ernst, D-4200 Oberhausen 14 (DE); Bahrmann, Helmut, Dr. Dipl.-Chem, D-4236 Hamminkeln/Brünen (DE)
(74) Vertreter: Reichelt, Karl-Heinz (DE)

## Beschreibung

Die Erfindung betrifft ein Verfahren zur Herstellung von Ethanol und n-Propanol aus Methanol und Synthesegas, d.h. dem Gemisch von Kohlenmonoxid und Wasserstoff in Gegenwart von Kobalt und Platin als Katalysatoren. Diese Reaktion, sie wird als Homologisierung bezeichnet, ermöglicht es, ausgehend von Methanol, durch Einführung einer oder mehrerer CH_{Z}-Gruppen höhere homologe Alkohole herzustellen.

Die Homologisierung findet im verstärkten Masse Interesse, da sie einen Weg zur Gewinnung höherer Alkohole eröffnet, der unabhängig von der Verwendung von Erdöl ist. Als Einsatzstoff wird Synthesegas bzw. daraus hergestelltes Methanol benötigt; Synthesegas ist, z.B. aus Kohle oder Erdgas nach verschiedenen, technisch bewährten Prozessen zugänglich.

Es ist seit langem bekannt (vgl. z.B. DE-PS 867849), Methanol mit Wasserstoff und Kohlenmonoxid in Gegenwart eines wasserlöslichen Kobaltkatalysators bei hohen Temperaturen und Drücken in Ethanol umzuwandeln. Die Reaktion verläuft nach folgender Summengleichung: 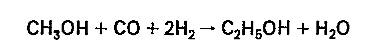 wobei in untergeordnetem Masse höhere Alkohole entsprechend 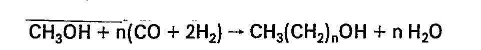 gebildet werden können.

Während ursprünglich für die Reaktion ausschliesslich Kobalt als Katalysator verwendet wurde, gewannen im Laufe der Zeit Mehrkomponenten-Katalysatoren zunehmend Bedeutung.

In der US-PS 3285948 ist die Herstellung von Ethanol aus Methanol unter Verwendung von Kobalt als Katalysator, Jod oder einer Jodverbindung als erstem und einem Rutheniumhalogenid oder einem Osmiumchlorid als weiterem Promotor beschrieben. Die beanspruchten Massnahmen sollen zu einer Erhöhung der Selektivität der Reaktion zu Ethanol führen.

Das gleiche Ziel wird nach dem Verfahren der DE-OS 2625627 mit einem Katalysatorsystem angestrebt, das aus Kobalt, einem Halogenid als Promotor und einem tertiären Phosphin besteht; die Umsetzung erfolgt in einem Kohlenwasserstoff als Lösungsmittel.

Nach der Lehre der US-PS 4133966 gewinnt man Ethanol aus Methanol und Synthesegas unter Verwendung eines aus Kobaltacetylacetonat, einer organischen Verbindung eines Elementes der Gruppe VA des Periodensystems der Elemente, einer Rutheniumverbindung und einerJodverbindung bestehenden Katalysators.

In der US-PS 3285948 wird u.a. berichtet, dass mit Platinverbindungen als Katalysatoren für die Homologisierungsreaktion keine deutlich besseren Ergebnisse erzielt werden als mit dem Katalysatorsystem Kobalt/Jod.

Trotz der vorstehend beschriebenen Massnahmen reicht die erzielte Selektivität der Reaktion für eine technische Anwendung nicht aus. Hierbei ist zu berücksichtigen, dass die Nebenprodukte nicht nur in grosser Menge, sondern in Form zahlreicher, unterschiedlicher Einzelverbindungen anfallen. So werden neben den erwünschten Alkoholen z.B. Methan, Ethan, Propan, verschiedene Ether sowie Methylacetat, Ethylacetat, Propylacetat, Acetaldehyddimethylacetal, Acetaldehyd-methylethylacetal und Acetaldehyd-diethylacetal gebildet. Bei industrieller Nutzung des Prozesses ist daher ein hoher Aufwand erforderlich, um die aus den Nebenprodukten gewinnbaren Wertproduktanteile, z.B. durch Hydrierung, Verseifung und Destillation, zu isolieren.

Eine Verbesserung der Selektivität der Umsetzung durch Zusatz eines Lösungsmittels zu den Reaktionspartnern hat eine erhebliche Abnahme des Umsatzes bezogen auf Reaktorvolumen und Zeit zur Folge.

Nach den bekannten Verfahren kann man also entweder Methanol mit zufriedenstellender Selektivität in höhere Alkohole überführen, erzielt dann aber nur geringe Umsätze, oder man erreicht hohe Umsätze bei niedriger Selektivität.

Es bestand daher die Aufgabe, eine Arbeitsweise zu entwickeln, die es erlaubt, Methanol in Ethanol und n-Propanol mit hoher Selektivität und hohem Umsatz zu überführen sowie die Anzahl der Nebenprodukte wesentlich zu reduzieren, so dass das Reaktionsgemisch in einfacher Weise aufgetrennt werden kann.

Die Erfindung löst diese Aufgabe mittels eines Verfahrens zur Herstellung von Ethanol und n-Propanol durch Umsetzung von Methanol mit Kohlenmonoxid und Wasserstoff bei erhöhten Drücken und Temperaturen von 150 bis 250°C in Gegenwart eines Katalysators, der Kobalt, Platin, mindestens ein Halogen oder Halogenid sowie ein organisches Phosphin oder Phosphit enthält. Es ist dadurch gekennzeichnet, dass das Halogen bzw. Halogenid Chlor oder ein Chlorid und das organische Phosphin oder Phosphit zweizähnig ist und die Reaktion bei Drücken von 200 bis 800 bar in Gegenwart von 5 bis 25 Gew.-% Wasser, bezogen auf Methanol, durchgeführt wird.

Kobalt wird dem Reaktionsgemisch im allgemeinen in Form eines Salzes wie Kobalt-2-ethyl- hexanoat, Kobaltacetylacetonat, Kobalthalogenid, Kobaltnitrat, als Oxid oder Hydroxid zugesetzt. Besonders bewährt hat sich Kobaltcarbonat. Es ist aber auch möglich, metallisches Kobalt in feinverteilter Form zu verwenden. Unter den Reaktionsbedingungen wandelt sich Kobalt bzw. die Kobaltverbindung mit Kohlenmonoxid und Wasserstoff unter Bildung eines Kobalt-Carbonyls oder -Hydrocarbonyls um.

Ein weiterer Bestandteil des Katalysatorsystems ist Platin, das dem Reaktionsgemisch als Verbindung, die sich unter den Reaktionsbedingungen im Reaktionsmedium löst, z.B. Platin-halogenid, Platin-2-äthylhexanoat, Platin-acetylacetonat, zugesetzt wird.

Wesentlich für das erfindungsgemässe Verfahren ist die Gegenwart von Chlor oder Chlorid während der Reaktion. Vorzugsweise verwendet man Chloride, insbesondere Chloride des Platins wie PtCl₂, PtCI₄ oder H₂PtCl₆.

Unter zweizähnigen Phosphinen oder Phosphiten, deren Einsatz kennzeichnend für die neue Arbeitsweise ist, werden solche Verbindungen verstanden, die zwei gleichzeitig als Donoren wirkende Phosphoratome enthalten.

Als organische Phosphine oder Phosphite, die im Rahmen der vorliegenden Erfindung eingesetzt werden können, haben sich besonders Verbindungen der allgemeinen Formel 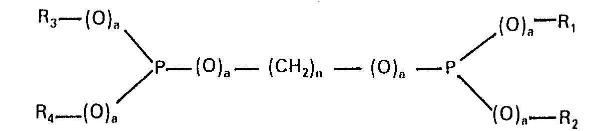 bewährt.

Hierbei sind Rₗ, R₂, R₃ und R₄ gleich oder verschieden und stehen für Wasserstoff, geradkettige oder verzweigte Alkylreste mit 1 bis 16 Kohlenstoffatomen, Cycloalkyl oder Arylreste mit 6 bis 15 Kohlenstoffatomen, a bedeutet 0 oder 1 und n die Zahlen 1, 2, 3, 4, 5, 6 oder 8.

Die Reste R, und R₂ bzw. R₃ und R₄ können auch miteinander verbunden sein und auf diese Weise einen Phosphor enthaltenden heterocyclischen Ring bilden.

Beispiele für Alkylreste sind Methyl, Ethyl, Propyl, Butyl, Pentyl, i-Propyl, i-Hexadecyl, Neopentyl, für Cycloalkylreste Cyclohexyl, Dicyclopentyl und für Arylreste Phenyl, Tolyl, Naphthyl, Phthalyl.

Die Reste R, und R₄ können über Sauerstoffatome mit dem Phosphoratom verbunden sein. Das gleiche gilt entsprechend der Bedeutung von a für die Verknüpfung beider Phosphoratome über den Alkylrest. Beispiele für Verbindungen entsprechend den beschriebenen Formeln, die im Rahmen der erfindungsgemässen Arbeitsweise eingesetzt werden, sind 1,3-Bis-(monophenyl- phosphino)-propan (1), 1,3-Bis-(diphenylphos- phino)-propan (2), Bis-(monophenylphosphino)-methan (3), Bis-(diphenylphosphino)-methan (4), 1,3-Bis-[1,3,2-dioxaphospholanyl-(2)]-propan (5), Bis-[1,3,2-dioxaphospholanyl-(2)]-methan (6), 1,3-Bis- (diphenoxyphosphino)-propan (7). 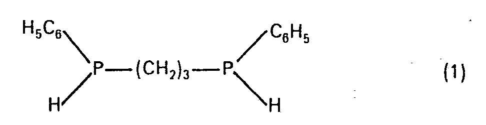 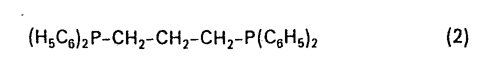 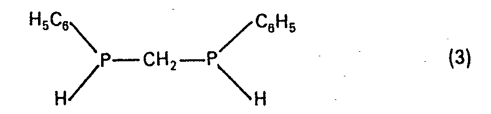 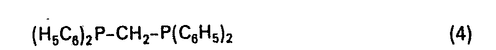 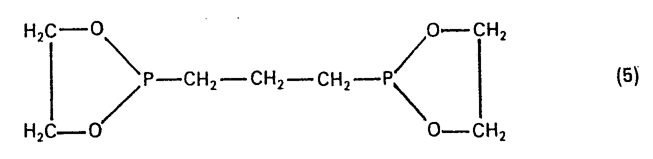 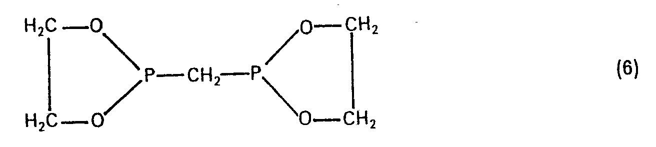 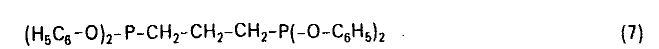 Die vorstehend beschriebenen Phosphorverbindungen bilden mit den Kobalt- und Platinverbindungen unter den Reaktionsbedingungen Komplexverbindungen, die ausserdem Kohlenmonoxid und Wasserstoff enthalten können. Diese Verbindungen stellen einen Bestandteil des wirksamen Katalysatorsystems dar.

Zweckmässig enthält das Katalysatorsystem neben Chlor oder Chlorid auch noch ein weiteres Halogen, vorzugsweise Jod in molekularer oder in ionischer Form. Als Salze des Jods können vorteilhaft Alkalimetalljodide und Kobaltjodid verwendet werden.

Das im erfindungsgemässen Verfahren eingesetzte Katalysatorsystem kann man dem Reaktionsgemisch in Form seiner einzelnen Bestandteile zuführen. Eine Präformierung der Metallkomplexverbindungen, die Komponenten des Katalysatorsystems sind, ist nicht erforderlich. Das Katalysatorsystem ist wiederholt einsetzbar.

Die Carbonyle bildenden Katalysatoren bzw. Katalysatorkomponenten können in einem hochsiedenden Lösungsmittel wie Diethylenglykolether (Diglyme), Tetraethylenglykolether (Tetraglyme), Neopentylglykol (2,2-Dimethyl-1,3-propandiol), Ethylenglykol, Trikresylphosphat suspendiert bzw. aufgelöst werden. Besonders geeignet als Lösungs- bzw. Suspensionsmittel sind die höher als Ethanol bzw. n-Propanol siedenden organischen Nebenprodukte der Reaktion.

Das als Ausgangsstoff eingesetzte Methanol kann in Form des in technischen Anlagen hergestellten Produktes mit einem Wassergehalt von 4-6% verwendet werden. Eine zusätzliche Reinigung ist nicht erforderlich.

Das ursprünglich eingesetzte Reaktionsgemisch enthält 5 bis 25 Gew.-% Wasser, bezogen auf Methanol. Durch den Wasserzusatz wird eine Erhöhung des Umsatzes bewirkt. Höhere Wassermengen beeinflussen den Umsatz nur unwesentlich, geringere Mengen haben keine oder nur unerhebliche Umsatzsteigerungen zur Folge. Zweckmässig wird das Wasser zusammen mit dem Methanol dem Reaktor zugeführt.

Das molare Verhältnis von Kobalt zu Methanol beträgt 1:20 bis 1:10000 insbesondere 1:30 bis 1:2000. Kobalt und Phosphin bzw. Phosphit werden in einem molaren Verhältnis von 1:0,1 bis 1:20 vorzugsweise 1:1 bis 1:5 eingesetzt.

Das Atomverhältnis von Kobalt zu Platin beträgt 1:0,0005 bis 1:1 vorzugsweise 1:0,01 bis 1:0,3.

Kobalt und Chlor bzw. Chlorid werden im molaren Verhältnis von 1:0,02 bis 1:2 insbesondere 1:0,05 bis 1:1 angewandt. Bei Einsatz eines weiteren Halogens bzw. Halogenids wird zweckmässigerweise dasselbe molare Verhältnis zwischen Kobalt und diesem zweiten Halogen bzw. Halogenid eingehalten.

Das Kohlenmonoxid-/Wasserstoff-Gemisch soll keine die Aktivität des Katalysatorsystems beeinflussenden Verunreinigungen wie Schwefel enthalten. Kohlendioxid und/oder Stickstoff bis zu 5 Vol.% bezogen auf das Gesamtgemisch schaden nicht.

Der neue Prozess kann sowohl diskontinuierlich als auch kontinuierlich durchgeführt werden. Im allgemeinen erfolgt die Umsetzung von Methanol, Kohlenmonoxid und Wasserstoff bei Temperaturen von 150 bis 250°C insbesondere 160 bis 230°C. Der Druck wird auf Werte zwischen 200 bis 800 bar vorzugsweise 450 bis 700 bar gehalten. Das molare Verhältnis von Wasserstoff zu Kohlenmonoxid im Synthesegas beträgt 1:1 bis 10:1.

Die nachstehend beschriebenen Beispiele erläutern die Erfindung.

### Beispiel 1

In einem Stahlautoklaven (1 I Inhalt), versehen mit Hubrührer, Temperaturmessung, Probenahmestutzen und einem Gasometer, das zum Auffangen gasförmiger Bestandteile dient, werden 6,25 mol (200 g) Methanol, 1,1 mol (19,80 g) Wasser, 17 mmol (2,02 g CoC0₃) Co, 6,7 mmol (1,00 g NaJ) Jodid, 22,1 mmol (9,11 g) 1,3-Bis-(diphenyl- phosphino)-propan und 0,5 mmol (0,168 g PtCl₄) Pt vorgelegt. Anschliessend wird mit Synthesegas (CO-H₂ = 1:3) ein Druck von 550 bar eingestellt, auf 185°C erhitzt und die Reaktion 6 Stunden lang unter Nachpressen von Synthesegas fortgesetzt.

Nach Abkühlen des Reaktionsgemisches und Entspannen in das Gasometer wird eine Durchschnittsprobe genommen, deren gaschromatographische Zusammensetzung in der Tabelle angegeben ist.

### Beispiel 2

Die in Beispiel 1 angefallene Reaktionsmischung wird destillativ von allen flüchtigen Bestandteilen befreit und der trockene Rückstand erneut in die Reaktion, wie in Beispiel 1 angegeben, mit 6,25 mol (200 g) Methanol und 1,1 mol (14,80 g) Wasser eingesetzt. Anschliessend wird mit Synthesegas (CO:H₂ = 1:3) ein Druck von 550 bar eingestellt, auf 185°C erhitzt und die Reaktion 3 Stunden lang unter Nachpressen von Synthesegas fortgesetzt.

Die Probenahme erfolgt wie in Beispiel 1 angegeben ist, die gaschromatographische Zusammensetzung der untersuchten Probe ist in der Tabelle angegeben.

### Vergleichsbeispiel 1

Es werden die gleichen Bedingungen wie in Beispiel 1 und 2 angegeben eingehalten, jedoch wird kein PtCI₄ eingesetzt. Die Reaktion wird wie in Beispiel 1 angegeben, durchgeführt.

Die Ergebnisse sind den folgenden Tabellen 1 und 2 zu entnehmen.

Die Gegenüberstellung der Beispiele 1 und 2 mit dem Vergleichsbeispiel 1 zeigt, dass der erfindungsgemässe Zusatz von Chlorid in Form von PtCI₄ Umsatz und Selektivität der Reaktion zu Ethanol und Propanol erhöht.

### Vergleichsbeispiel 2

Es werden die gleichen Bedingungen wie in Beispiel 1 angegeben eingehalten, jedoch wird anstelle von PtCI₄ PtJ₂ eingesetzt. Die Gegenüberstellung der Beispiele 1 und 2 mit dem Vergleichsbeispiel 2 zeigt, dass PtCI₄ eine besonders geeignete Form der Platinzugabe ist.

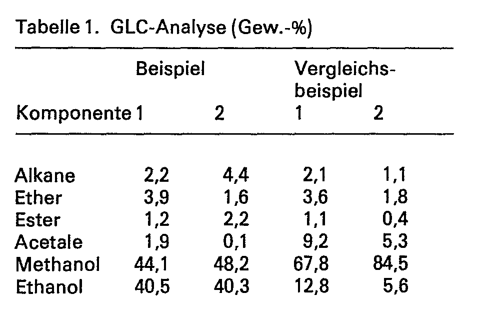

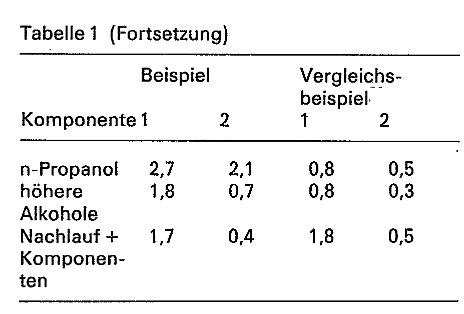

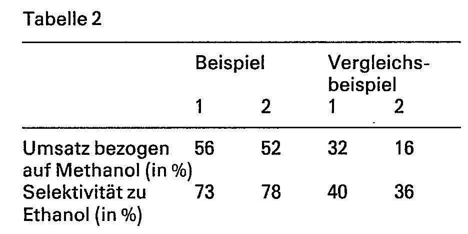

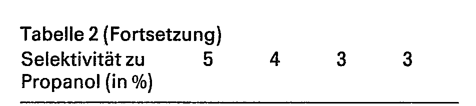

## Patentansprüche

1. Verfahren zur Herstellung von Ethanol und n-Propanol durch Umsetzung von Methanol mit Kohlenmonoxid und Wasserstoff bei erhöhten Drücken und Temperaturen von 150 bis 250°C in Gegenwart eines Katalysators, der Kobalt, Platin, mindestens ein Halogen oder Halogenid sowie ein organisches Phosphin oder Phosphit enthält, dadurch gekennzeichnet, dass das Halogen bzw. Halogenid Chlor oder ein Chlorid und das organische Phosphin oder Phosphit zweizähnig ist und die Reaktion bei Drücken von 200 bis 800 bar in Gegenwart von 5 bis 25 Gew.-% Wasser, bezogen auf Methanol, durchgeführt wird.

2. Verfahren nach Anspruch 1, dadurch gekennzeichnet, dass als zweizähniges organisches Phosphin oder Phosphit Verbindungen der allgemeinen Formel 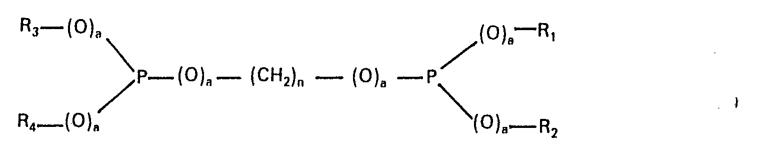 verwendet werden
wobei R₁, R₂, R₃ und R₄ gleich oder verschieden sind und für Wasserstoff, geradkettige oder verzweigte Alkylreste mit 1 bis 16 Kohlenstoffatomen, Cycloalkyl- oder Arylreste mit 6 bis 15 Kohlenstoffatomen stehen, a 0 oder 1 und n die Zahlen 1, 2, 3, 4, 5, 6 oder 8 bedeuten.

3. Verfahren nach Anspruch 2, dadurch gekennzeichnet, dass die Reste R, und R₂ bzw. R₃ und R₄ miteinander verbunden sind.

4. Verfahren nach Anspruch 1 bis 3, dadurch gekennzeichnet, dass das Halogenid PtCl₂, PtCI₄ oder H₂PtCl₆ ist.

5. Verfahren nach Anspruch 1 bis 4, dadurch gekennzeichnet, dass das molare Verhältnis von Kobalt zu Methanol 1:20 bis 1:10000 vorzugsweise 1:30 bis 1:2000 und das molare Verhältnis von Kobalt zu Phosphin bzw. Phosphit 1:0,1 bis 1:20 vorzugsweise 1:1 bis 1:5 beträgt.

6. Verfahren nach Anspruch 1, dadurch gekennzeichnet, dass das Atomverhältnis von Kobalt zu Platin 1:0,0005 bis 1:1, vorzugsweise 1:0,01 bis 1:0,3 beträgt.

7. Verfahren nach Anspruch 1, dadurch gekennzeichnet, dass das molare Verhältnis von Kobalt zu Chlor 1:0,02 bis 1:2, vorzugsweise 1:0,05 bis 1:1 beträgt.

## Claims

1. Process for producing ethanol and n-propanol by converting methanol with carbon monoxide and hydrogen at elevated pressures and temperatures of 150 to 250°C in the presence of a catalyst containing cobalt, platinum, at least one halogen or halide, as well as an organic phosphine or phosphite, characterised in that the halogen or halide is chlorine or a chloride and the organic phosphine or phosphite is bi-dentate and the reaction is carried out at pressures of 200 to 800 bars in the presence of 5 to 25% by weight of water referred to methanol.

2. Process according to claim 1, characterised in that as bi-dentate organic phosphine or phosphite, compounds of the general formula 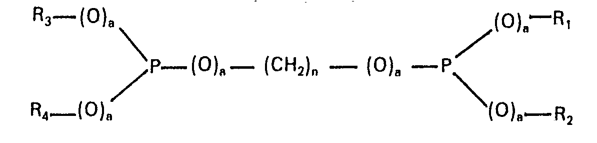 are used, wherein R₁, R₂, R₃ and R₄ are the same or different and denote hydrogen, straight chain or branched alkyl radicals with 1 to 16 carbon atoms, cycloalkyl or aryl radicals with 6 to 15 carbon atoms, a denotes 0 or 1, and n denotes the numbers 1,2,3,4,5,6 or 8.

3. Process according to claim 2, characterised in that the radicals R, and R₂, and R₃ and R₄ are joined to one another.

4. Process according to claims 1 to 3, characterised in that the halide is PtCl₂, PtCI₄ or H₂PtCl₆.

5. Process according to claims 1 to 4, characterised in that the molar ratio of cobalt to methanol is 1:20 to 1:10,000, preferably 1:30 to 1:2000, and the molar ratio of cobalt to phosphine or phosphite is 1:0.1 to 1:20, preferably 1:1 to 1:5.

6. Process according to claim 1, characterised in that the atomic ratio of cobalt to platinum is 1:0.0005 to 1:1, preferably 1:0.01 to 1:0.3.

7. Process according to claim 1, characterised in that the molar ratio of cobalt to chlorine is 1:0.02 to 1:2, preferably 1:0.05 to 1:1.

## Revendications

1. Procédé de préparation de l'éthanol et du n-propanol par réaction du méthanol avec le mo- noxyde de carbone et l'eau sous des pressions élevées et à des températures de 150 à 250°C en présence d'un catalyseur qui contient du cobalt, du platine, au moins un halogène ou halogénure ainsi qu'une phosphine ou un phosphite organique, procédé caractérisé en ce que l'halogène ou l'halogénure est le chlore ou un chlorure, la phosphine ou le phosphite organique est bidenté et l'on effectue la réaction sous des pressions de 200 à 800 bars en présence de 5 à 25% en poids d'eau, par rapport au méthanol.

2. Procédé selon la revendication 1, caractérisé en ce qu'on utilise comme phosphine ou phosphite organique bidenté des composés de formule générale 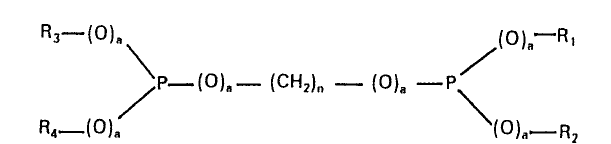 dans laquelle R,, R₂, R₃ et R₄, sont identiques ou différents, et représentent de l'hydrogène, des. radicaux alkyles linéaires ou ramifiés comportant 1 à 16 atomes de carbone, des radicaux cycloal- kyles ou aryles comportant 6 à 15 atomes de carbone; a est nul ou vaut 1 et n représente les nombres 1,2,3,4,5,6 ou 8.

3. Procédé selon la revendication 2, caractérisé en ce que les radicaux R, et R₂ ou R₃ et R₄ sont reliés entre eux.

4. Procédé selon l'une quelconque des revendications 1 à 3, caractérisé en ce que l'halogénure est PtCl₂, PtC1₄ ou H₂PtCl₆.

5. Procédé selon l'une des revendications 1 à 4, caractérisé en ce que le rapport molaire du cobalt ou méthanol se situe entre 1:20 et 1:10000, avantageusement entre 1:30 et 1:2000, et le rapport molaire du cobalt à la phosphine ou au phosphite se situe entre 1:0,1 et 1:20, avantageusement entre 1:1 et 1:5.

6. Procédé selon la revendication 1, caractérisé en ce que le rapport atomique du cobalt au platine se situe entre 1:0,0005 et 1:1, avantageusement entre 1:0,01 et 1:0,3.

7. Procédé selon la revendication 1, caractérisé en ce que le rapport molaire du cobalt au chlore se situe entre 1:0,02 et 1:2, avantageusement entre 1:0,05 et 1:1.
